(19) Europäisches Patentamt
European Patent Office
Office européen des brevets

(11) Veröffentlichungsnummer : **0 049 858**
**B1**

(12) # EUROPÄISCHE PATENTSCHRIFT

(45) Veröffentlichungstag der Patentschrift : **20.02.85**

(21) Anmeldenummer : **81108007.6**

(22) Anmeldetag : **07.10.81**

(51) Int. Cl.[4] : **C 07 D211/46**, C 07 D211/02, C 07 H 5/06, C 12 P 19/26// A61K31/445

(54) **Verfahren zur Herstellung von N-substituierten Derivaten des 1-Desoxynojirimycins.**

(30) Priorität : **15.10.80 DE 3038901**

(43) Veröffentlichungstag der Anmeldung : **21.04.82 Patentblatt 82/16**

(45) Bekanntmachung des Hinweises auf die Patenterteilung : **20.02.85 Patentblatt 85/08**

(84) Benannte Vertragsstaaten : **AT BE CH DE FR GB IT LI NL SE**

(56) Entgegenhaltungen :
**EP-A- 0 000 947**
**EP-A- 0 007 040**
**EP-A- 0 009 633**
**EP-A- 0 012 278**
**DE-A- 2 658 563**
**DE-A- 2 834 122**
**DE-A- 2 846 118**

(73) Patentinhaber : **BAYER AG**
**Konzernverwaltung RP Patentabteilung**
**D-5090 Leverkusen 1 Bayerwerk (DE)**

(72) Erfinder : **Kinast, Günther, Dr.**
**Am Eckbusch 42**
**D-5600 Wuppertal 1 (DE)**
Erfinder : **Schedel, Michael, Dr.**
**Sillerstrasse 49**
**D-5600 Wuppertal 11 (DE)**
Erfinder : **Koebernick, Wolfgang, Dr.**
**Claudiusweg 3**
**D-5600 Wuppertal 11 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 049 858 B1

**Beschreibung**

Die Erfindung betrifft ein neues Verfahren zur Herstellung von Verbindungen der Formel I

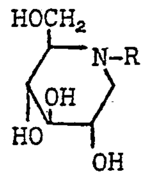

(I)

worin

R Wasserstoff, gegebenenfalls substituiertes Alkyl oder Aralkyl bedeutet.

Es ist bekannt, daß die Verbindungen der Formel I sehr gute α-Glucosidaseinhibitoren sind und als Mittel gegen Diabetes geeignet sind (siehe EP-A1-0 000 947).

Aus der DE-OS 28 34 122 ist ein Verfahren zur Herstellung von 1-Desoxynojirimycin I (R=H) bekannt, bei dem 1-Aminosorbit II mikrobiologisch zu 6-Aminosorbose III oxidiert wird, die dann zu 1-Desoxynojirimycin I (R=H) hydriert wird. Dieses Verfahren ist jedoch hinsichtlich der Ausbeuten, insbesondere

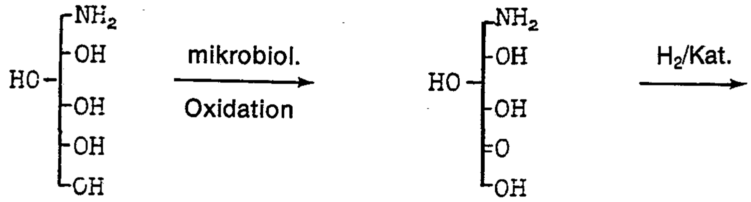


(R=H) (I)

(II) (III)

der Volumenausbeuten bei der mikrobiologischen Umsetzung noch nicht optimal.

Aus der EP-AZ-0 012 278 ist bekannt, daß man die Verbindungen der Formel I erhält, wenn man die Aminosorbite der Formel IV mit einer hydrogenolytisch abspaltbaren und im anschließenden mikrobiologischen Oxidationsverfahren beständigen Schutzgruppe schützt, die so erhaltenen Verbindungen V mikrobiologisch zu den geschützten 6-Aminosorbosen VI oxidiert und anschließend durch Hydrogenolyse die Schutzgruppe abspaltet und den Ring zu den Verbindungen der Formel I schließt.

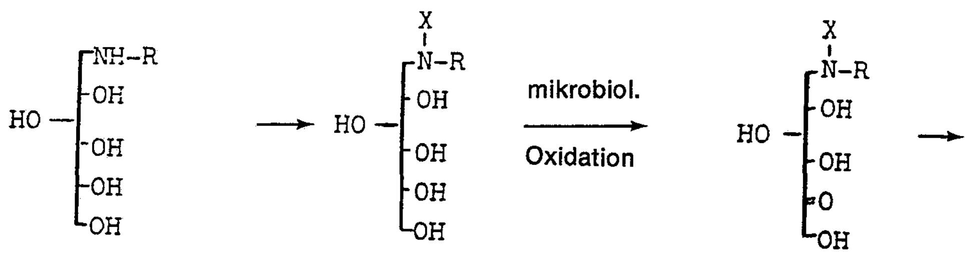


(IV) (V) (VI)

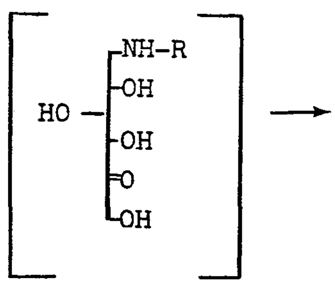

(I)

(VII)

Hierbei wird jedoch bei dem Schritt der Hydrierung eine relativ große Menge Katalysator benötigt. Außerdem sind bei diesem Verfahren die als Zwischenprodukte entstehenden 6-Amino-sorbosen VII nicht in Substanz isolierbar.

Verbindungen der Formel VII sind wichtig als α-Glucosidaseinhibitoren (DE-OS 28 30 457, DE-OS 28 30 424) und als Zwischenprodukte für die Herstellung von Desoxynojirimycinen der Formel IX (EP-A1-0 009 633).

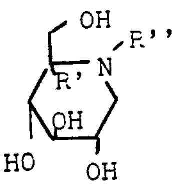

(IX)

Weitere Verfahren zur Herstellung der Verbindungen der Formel I sind in der EP-A2-0 012 278 zitiert sowie in der EP-A1-0 000 947 aufgeführt. Die dort beschriebenen synthetischen Verfahren verlaufen alle über mehrere zeitraubende Stufen, in denen aufwendige Reinigungsschritte notwendig sind.

Es wurde nun gefunden, daß man die Verbindungen der Formel I auf einem einfachen Wege und in hohen Ausbeuten erhalten kann, wobei die 6-Amino-sorbosen VII als Zwischenprodukte mit hoher Reinheit isoliert werden können und wobei die Notwendigkeit des Einsatzes großer Katalysatormengen nicht besteht.

Die Erfindung betrifft daher ein Verfahren zur Herstellung von Desoxynojirimycin und seinen Derivaten der Formel I, dadurch gekennzeichnet, daß man Glucose X in an sich bekannter Weise in die 1-Aminosorbite IV überführt, wobei R die unter Formel I genannte Bedeutung aufweist, die Aminogruppe mit einer sauer abspaltbaren und im sich anschließenden mikrobiologischen Oxidations-verfahren beständigen Schutzgruppe schützt, die so dargestellten Verbindungen XI auf an sich bekannte Weise mikrobiologisch zu den geschützten 6-Aminosorbose XII oxidiert, anschließend die Schutzgruppe sauer abspaltet und die so erhaltenen Salze der 6-Aminosorbosen VII entweder nach ihrer Isolierung oder in einem Arbeitsgang in an sich bekannter Weise zu den Verbindungen

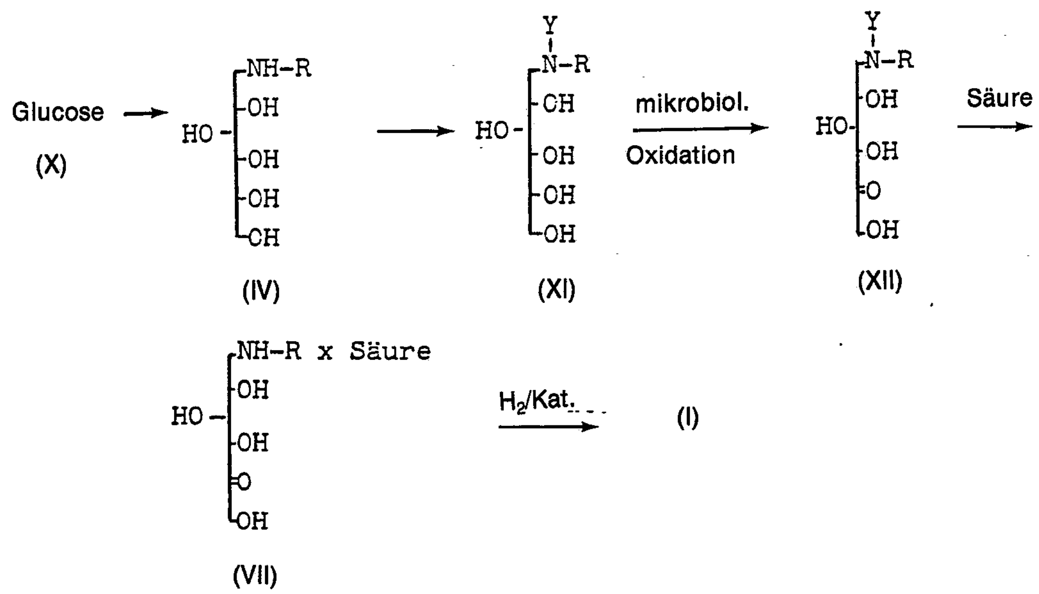


der Formel I hydriert.

Der Rest R bedeutet vorzugsweise Wasserstoff oder gegebenenfalls durch OH oder $C_1$-$C_4$-Alkoxy oder durch gegebenenfalls substituiertes Phenyl substituiertes $C_1$-$C_{10}$-Alkyl.

Ganz besonders bevorzugt bedeuten R Wasserstoff, $C_1$-$C_{10}$-Alkyl oder Hydroxyethyl.

Als Schutzgruppe Y, die sich sauer abspalten lässt und bei der mikrobiologischen Oxidation beständig ist, eignen sich z. B. der tert.-Butyloxycarbonyl-, der Formyl-, der Dichloroacetyl- oder der o-Nitrosulfenylrest ; aber auch andere Schutzgruppen aus der Vielzahl der sauer abspaltbaren Schutzgruppen sind prinzipiell für die Durchführung des erfindungsgemäßen Verfahrens geeignet. Ganz besonders bevorzugt sind tert.-Butyloxycarbonyl oder o-Nitrosulfenyl.

Die tert.-Butyloxycarbonyl (BOC)-Schutzgruppe wird in an sich bekannter Weise z. B. mit Di-BOCpyrocarbonat z. B. in einem Gemisch aus Wasser und einem mit Wasser mischbaren Lösungsmittel in die Verbindungen der Formel IV eingeführt.

Die mikrobiologische Oxidation der Verbindungen der Formel XI zu den Verbindungen der Formel XII wird in an sich bekannter Weise wie in der EP-A2-0 012 278 beschrieben durchgeführt. Hierbei ist es günstig, die zu oxidierende Verbindung der Formel XI mehrmals der bakteriellen Kulturbrühe zuzufüttern jeweils nach vollständiger Oxidation oder vorangegangenen Zugabe. Dadurch können besonders hohe Volumenausbeuten erzielt werden können.

Mikroorganismen, die zur Durchführung der Oxidation geeignet sind oder aus denen aktive Extrakte zur Durchführung der Oxidation gewonnen werden können, können Prokaryonten, also Bakterien oder Eukaryonten, z. B. Pilze, sein und jeweils den verschiedensten taxonomischen Gruppen angehören. Der Fachmann

auf mikrobiologischem Gebiet kann geeignete Mikroorganismen leicht finden, indem er eine größere Zahl aerober oder fakultativ aerober Mikroorganismen in einem entsprechenden Nährmedium, das eine Verbindung der Formel XI enthält, kultiviert und auf die Fähigkeit überprüft, die erfindungsgemäße Oxidationsreaktion zu katalysieren und Verbindungen der Formel XII anzuhäufen.

Insbesondere können für die Oxidation z. B. Bakterien der Ordnung Pseudomonadales, innerhalb dieser Ordnung besonders Vertreter der Familie Pseudomonadaceae und hier vor allen Dingen Bakterien der Gattung Gluconobacter eingesetzt werden. Ferner haben sich auch Bakterien aus der Gruppe der coryneformen Bakterien, besonders solche der Gattung Corynebaceterium als geeignet erwiesen. Schließlich kann die Oxidation auch mit Pilzen, so z. B. mit Hefen der Ordnung Endomycetales, besonders mit solchen der Familie Spermophthoraceae und hier hauptsächlich mit Vertretern der Gattung Metschnikowia durchgeführt werden.

Als Beispiele seien genannt :

Gluconobacteroxidans spp. suboxydans (DSM 50 049), Glucobacteroxidans spp. soboxydans (DSM 2003), Corynebacterium betae (DSM 20 141) und Metschnikowia pulcherima (ATCC 20 515).

Die DSM-Nummern geben die Nummern an, unter denen die genannten Mikroorganismen bei der Deutschen Sammlung für Mikroorganismen, Göttingen, hinterlegt sind. Metschnikowia pulcherrima ist bei der American Type Culture Collection, Rockville Maryland USA, hinterlegt.

Wird die Oxidation mit intakten Mikroorganismen in wachsender Kultur durchgeführt, können feste, halbfeste oder flüssige Nährmedien verwendet werden. Bevorzugt werden wäßrig-flüssige Nährmedien herangezogen.

Die Kultur kann in allen Nährmedien durchgeführt werden, welche bekannterweise zur Kultivierung von Mikroorganismen der obengenannten Gruppen verwendet werden und die die zu oxidierende Verbindung enthalten. Das Nährmedium muß assimilierbare Kohlenstoff- und Stickstoffquellen, sowie Mineralsalze enthalten. Als assimilierbare Kohlenstoff- und Stickstoffquellen eignen sich vor allem komplexe Gemische wie sie insbesondere biologische Produkte verschiedenen Ursprungs darstellen, z. B. Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt. Als Stickstoffquellen kommen zusätzlich Ammoniumsalze und Nitrate, z. B. Ammoniumchlorid, Ammoniumsulfat, Natriumnitrat und Kaliumnitrat, in Frage. Die Mineralsalze, welche im Nährmedium enthalten sein sollen, liefern z. B. folgenden Ionen : $Mg^{++}$, $Na^{+}$, $K^{+}$, $Ca^{++}$, $NH_4^{+}$, $Cl^{-}$, $SO_4^{--}$, $PO_4^{---}$ und $NO_3^{-}$ sowie Ionen der üblichen Spurenelemente wie Cu, Fe, Mn, Mo, Zn, Co und Ni.

Falls in den genannten komplexen Nährbodenbestandteilen oder im verwendeten Wasser diese Salze bzw. Spurenelemente nicht ausreichend vorhanden sind, ist es zweckmäßig, das Nährmedium entsprechend zu ergänzen.

Es hat sich gezeigt, daß durch Zusatz von Verbindungen des Intermediärstoffwechsels sowie von anderen Zellbausteinen zum Medium, so z. B. von Aminosäuren oder von Verbindungen aus dem Tricarbonsäurezyklus eine wesentliche Verkürzung der Umsetzung ermöglicht wird.

Die zu oxidierende Verbindung kann entweder allein oder im Gemisch mit einer oder mehreren oxidierbaren Verbindungen dem Grundnährmedium zugesetzt werden. Als zusätzliche oxidierbare Verbindungen können primäre Alkohole, beispielsweise Ethanol, sekundäre Alkohole, beispielsweise Isopropanol, Polyole, beispielsweise Sorbit oder Glycerin, Aldehyde, beispielsweise Glykolaldehyd, Aldosen, beispielsweise Glucose oder Gluconsäuren verwendet werden.

Wird der Nährlösung eine oder mehrere der genannten Verbindungen zugesetzt, kann die zu oxidierende Verbindung entweder vor dem Animpfen oder an einem beliebigen späteren Zeitpunkt zwischen früher lag-Phase und später stationärer Wachstumsphase zugesetzt werden. In einem solchen Fall wird der betreffende Organismus auf den jeweils zugesetzten oxidierbaren Verbindungen vorkultiviert.

Für die Oxidation ist ein pH-Bereich zwischen 2 und 10, vorzugsweise zwischen 4 und 8, geeignet. Es ist günstig, die Kultur in einem Bereich zu puffern, beispielsweise mit Phosphat- oder Acetatpuffer.

Wie in der Fermentationstechnologie üblich, kann auch eine automatische pH-Regulierung durchgeführt werden, bei der sterile organische oder anorganische Säure, beispielsweise Schwefelsäure, oder sterile Lauge, beispielsweise Natronlauge, in Abständen in die Kulturlösung eingespritzt wird.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Kulturgefäße können z. B. die Dampf- als auch die Trockensterilisation angewendet werden ; Luft und die Kulturmedien können ebenfalls durch Dampf, aber auch durch Filtration sterilisiert werden.

Die Beimpfung der Nährmedien erfolgt nach allgemein üblichen Methoden, z. B. über Schrägröhrchen- oder Kolbenkulturen. Die Kultur erfolgt unter aeroben Bedingungen und kann gemäß den allgemein üblichen Methoden, beispielsweise unter Verwendung von Schüttelkulturen z. B. in Schüttelkolben, von luftbewegten Kulturen oder von Submerskulturen durchgeführt werden. Bevorzugt erfolgt die Kultivierung im aeroben Submersverfahren in belüfteten Fermentern z. B. in üblichen Submersfermentationstanks. Es ist möglich, die Kultur kontinuierlich oder diskontinuierlich durchzuführen. Vorzugsweise wird diskontinuierlich gearbeitet.

Es ist zweckmäßig, sicherzustellen, daß die Mikroorganismen ausreichend mit Sauerstoff sowie den

Nährstoffen in Kontakt gebracht werden. Dies kann nach der allgemein üblichen Methode wie Schütteln und Rühren, erfolgen.

Falls bei der Kultivierung in unerwünschter Menge Schaum entsteht, können die üblichen chemischen Schaumdämpfungsmittel, z. B. flüssige Fette und Öle, Öl-Wasser-Emulsionen, Paraffine, höhere Alkohole, wie Octadecanol, Siliconöle, Polyoxiethylen- bzw. Polyoxipropylen-Verbindungen zugesetzt werden. Schaum kann auch mit Hilfe der üblichen mechanischen Vorrichtungen gedämpft oder beseitigt werden.

Die Züchtungstemperatur kann zwischen etwa 20 und etwa 45 °C liegen. Die Dauer der Züchtung kann stark variiert werden, wobei z. B. die Zusammensetzung des Nährmediums und die Züchtungstemperatur eine Rolle spielen.

Die jeweiligen optimalen Bedingungen können von jedem Fachmann auf dem mikrobiologischen Gebiet leicht festgelegt werden.

Es hat sich herausgestellt, daß zur vollständigen Umsetzung von den der Kulturbrühe zugegebenen zu oxidierenden Verbindungen im allgemeinen eine Inkubationsdauer von zwischen 3 Stunden und 7 Tagen nach Zugabe notwendig ist.

Es ist auch möglich, die Oxidationsreaktion mit konzentrierten Zellsuspensionen geeigneter Mikroorganismen durchzuführen. Konzentrierte Zellsuspensionen werden wie folgt hergestellt : Die in Frage kommenden Mikroorganismen werden in einer geeigneten Nährlösung kultiviert, dann z. B. durch Zentrifugation geerntet und in einem kleineren Volumen derselben Nährlösung oder in Salz- oder Pufferlösungen, z. B. physiologische Kochsalzlösung, wäßrigen Lösungen von $KH_2PO_4$, Na-Acetat, -Maleinat oder einfach in Leitungs- oder destilliertem Wasser, suspendiert. Einer solchen Zellsuspension werden dann die zu oxidierenden Verbindungen zugesetzt und die Oxidationsreaktion unter den oben für wachsende Kulturen beschriebenen Bedingungen durchgeführt.

Der Vorteil dieses Verfahrens ist die durch die höhere Mikroorganismen-Konzentration ermöglichte Verkürzung der Reaktionsdauer der Oxidation auf wenige Stunden.

Es ist weiterhin möglich, die Oxidation nicht nur mit wachsenden Kulturen von Mikroorganismen oder mit daraus gewonnenen konzentrierten Zellsuspensionen durchzuführen, sondern auch mit aus diesen Bakterien hergestellten Extrakten oder Extraktfraktionierungen. Dabei kann es sich um Rohextrakte handeln, wie sie durch herkömmlichen Aufschluß von Mikroorganismenzellen gewonnen werden. Als Aufschlußmethoden können dienen : Ultraschallbehandlung, Passage durch eine French-Druckzelle, Zerreiben mit Quarzsand, Inkubation mit lysierenden Enzymen, Autolyse oder mehrfaches Einfrieren und Auftauen.

Werden nichtfraktionierte Rohextrakte zur Oxidation verwandt, haben sich prinzipiell dieselben Reaktionsbedingungen als günstig erwiesen, wie sie für die Durchführung der Oxidation mit wachsenden oder ruhenden Mikroorganismenzellen beschrieben wurden.

Soll die Oxidation mit teilweise gereinigten Extraktpräparationen (Enzymen) durchgeführt werden, so können zur Gewinnung derartiger Präparationen die allgemein üblichen Methoden der Proteinchemie angewandt werden, wie Ultrazentrifugation, Fällungsreaktion, Ionenaustausch- oder Adsorptionschromatographie, Gelfiltration oder elektrophoretische Methoden. Zur Klärung der Frage, welche von mehreren durch eine der genannten Methoden gewonnenen Fraktionen zur Katalyse der erfindungsgemäßen Oxidationsreaktion geeignet ist, wird ein Aliquot dieser Fraktion bei einer Temperatur zwischen 20 und 45 °C und einem pH zwischen 2 und 10 mit der zu oxidierenden Verbindung gemischt und der Ansatz auf die Bildung des Reaktionsprodukts dünnschichtchromatographisch untersucht. Zur Durchführung der Reaktion mit fraktionierten Zellextrakten kann es notwendig sein, daß zusätzliche Reaktionskomponenten dem Ansatz zugefügt werden müssen, z. B. physiologische oder künstliche Elektronenakzeptoren etwa $NAD^+$, $NADP^+$, Methylenblau, Dichlorphenolindophenol, Tetrazoliumsalze etc. Müssen solche zusätzlichen Reaktionskomponenten zugefügt werden, können diese entweder in Substratmengen, also in Konzentrationen, die der der eingesetzten, zu oxidierenden Verbindung entsprechen, oder in katalytischen Mengen, d. h. in Konzentrationen, die deutlich unter der gewählten Konzentration der zu oxidierenden Verbindung liegen, eingesetzt werden.

Soll im zweiten Fall eine annähernd quantitative Durchführung der Oxidation gewährleistet sein, so ist dem Reaktionsansatz weiterhin ein System zuzufügen, das die nur in katalytischer Menge vorhandene Reaktionskomponente ständig regeneriert. Es kann sich dabei z. B. um ein Enzym handeln, das für die Reoxidation eines im Verlaufe der Oxidation reduzierten Elektronenakzeptors in Gegenwart von Sauerstoff oder anderen Oxidationsmitteln sorgt.

Ansonsten haben sich auch für die Durchführung der Oxidation mit fraktionierten Zellextrakten dieselben Bedingungen als günstig erwiesen, wie sie oben für die Oxidation in wachsenden Mikroorganismenkulturen oder konzentrierten Zellsuspensionen angegeben wurden. Insbesondere gilt auch hier ein Temperaturbereich von 20 bis 45 °C und ein pH-Bereich von 2 bis 10. Die Menge der gebildeten Reaktionsprodukte erreicht jedoch in einem kürzeren Zeitraum ihr Maximum. Je nach Extraktkonzentration genügt eine Inkubationszeit zwischen 2 Stunden und 3 Tagen.

Die zeitabhängige Bildung der gewünschten Verbindung im Kulturmedium kann dünnschichtchromatographisch verfolgt werden.

Die Verbindungen der Formel XII werden in an sich bekannter Weise wie z. B. in der EP-A2-0 012 278 beschrieben, isoliert. Es ist besonders vorteilhaft, die Verbindungen der Formel XII direkt aus dem

Oxidationsmedium auskristallisieren zu lassen, abzutrennen und aus einem geeigneten Lösungsmittel, z. B. einem Alkohol wie Methanol, umzukristallisieren.

Zur Abspaltung der Schutzgruppen rührt man die entsprechenden blockierten 6-Amino-L-sorbosen XII bei Raumtemperatur in konzentrierter oder verdünnter Säure (z. B. 8N Salzsäure) und verfolgt den Ablauf der Abspaltung dünnschichtchromatographisch. Nach Zugabe von mit Wasser mischbaren organischen Lösungsmitteln (z. B. n-Propanol) kann man die deblockierten 6-Amino-L-sorbosen VII als Hydrochlorid in kristalliner Form oder als Sirup isolieren. Die so erhaltenen Salze der Verbindungen der Formel VII werden in an sich bekannter Weise (siehe EP-A1-0 007 040) durch Hydrieren in die Verbindungen der Formel I überführt.

Die Hydrierung erfolgt in wäßriger Lösung bei Raumtemperatur und einen $H_2$-Überdruck (beispielsweise $20 \times 10^5$ Pa). Als Katalysatoren eignen sich Edelmetallkatalysatoren (beispielsweise 5 %ige Pt-Kohle), vorteilhafter jedoch wird Ra-Nickel eingesetzt.

## Beispiele

### Beispiel 1

Herstellung von 1-tert.-Butyloxycarbonylaminosorbit

90 g 1-Aminosorbit werden in einem Gemisch aus 500 ml Tetrahydrofuran und 500 ml Wasser vorgelegt und 131 g Di-tert.-butyloxycarbonylpyrocarbonat in 500 ml Tetrahydrofuran bei Raumtemperatur hinzugetropft. Man rührt über Nacht, dampft das Tetrahydrofuran im Vakuum ab, extrahiert die wäßrige Phase 2 × mit Essigester, dampft anschließend die wäßrige Phase zur Trockene ein und kristallisiert den Rückstand aus iso-Propanol um.

Ausbeute : 110 g, Fp. : 86-88 °C.

### Beispiel 2

Herstellung von 1-tert.-Butyloxycarbonylamino-N-methylsorbit

Die Herstellung erfolgte analog Beispiel 1 aus 1-Methylaminosorbit.
Ausbeute : 95 % ; Fp. : 78-80 °C.

### Beispiel 3

Herstellung von 1-tert.-Butyloxycarbonylamino-N-(β-hydroxyethyl)-sorbit

Die Herstellung erfolgte analog Beispiel 1 aus 1-β-Hydroxyethylaminosorbit.
Ausbeute : 78 % ; Fp. : 103-104 °C.

### Beispiel 4

Oxidation von 1-tert.-Butyloxycarbonylaminosorbit durch Gluconobacter oxidans ssp. suboxidans (DSM 50 049).

Der Umsatz erfolgte in folgender Nährlösung :

20 g/l Hefeextrakt
50 g/l Sorbit
4 g/l $KH_2PO_4$

Die Nährlösungsbestandteile wurden in entmineralisiertem Wasser gelöst, der pH-Wert wurde mit NaOH auf 6,5 eingestellt. Die Nährlösung wurde zu je 250 ml in 1 Lit. Erlenmeyerkolben abgefüllt und 20 Min. bei 121 °C autoklaviert. Nach dem Abkühlen wurden 10 g/l 1-tert.-Butyloxicarbonylaminosorbit steril zugegeben und die Nährlösung 5 %ig mit einer Vorkultur, die in der selben Nährlösung, jedoch ohne 1-tert.-Butyloxicarbonylaminosorbit, gezüchtet worden war, beimpft. Die Inkubation erfolgte bei 28 °C auf einer Rundschüttelmaschine bei 280 Upm. Der Umsatz wurde dünnschichtchromatographisch verfolgt.

Nach 96 Stunden war vollständiger Umsatz von 10 g/l erzielt. Zur Isolierung der 6-tert.-Butyloxycarbonylaminosorbose wurde der Fermentationsansatz mit Butanol extrahiert, der Extrakt einrotiert und der Rückstand aus Essigester umkristallisiert.

Fp. : 141-143 °C.

### Beispiel 5

Oxidation von 1-tert.-Butyloxycarbonylaminosorbit durch Metschnikowia pulcherrima (ATCC 20 515)

Der Mikroorganismus Metschnikowia pulcherrima (ATCC 20 515) wurde in Schrägröhrchen auf einem Medium vorkultiviert, das pro 1 Lit. 3 g Hefeextrakt, 6 g Pepton, 10 g Glucose, 8 g NaCl und 20 g Agar in entmineralisiertem Wasser enthielt. Mit dieser Vorkultur wurden 250 ml Flüssigmedium (in einem 1 Lit. Erlenmeyerkolben) beimpft, das in 1 Lit. 3 g Hefeextrakt, 6 g Pepton, 10 g Sorbit, 8 g NaCl und 10 g 1-tert.-Butyloxycarbonylaminosorbit gelöst in entmineralisiertem Wasser enthielt und im Autoklaven durch Erhitzen für 20 Minuten auf 121 °C sterilisiert worden war. Die Kultur wurde bei 35 °C auf einer Rundschüttelmaschine bei 200 Upm inkubiert. Der Gehalt an 1-tert.-Butyloxycarbonylaminosorbose in der Kulturbrühe wurde dünnschichtchromatographisch bestimmt. Nach 2 Tagen waren 3 g/l (30 %) umgesetzt. Die Fermentation wurde zu diesem Zeitpunkt abgebrochen.

## Beispiel 6

Oxidation von 1-tert.-Butyloxycarbonylaminosorbit durch Corynebacterium betae (DSM 20 141)

Der Mikroorganismus Corynebacterium betae (DSM 20 141) wurde auf Schrägröhrchen vorkultiviert, die folgenden Nährböden enthielten : 10 g/l tryptisch verdautes Casein-Pepton, 6 g/l Hefeextrakt, 5 g/l Sorbit, 5 g/NaCl und 20 g/l Agar in entmineralisiertem Wasser. Mit einem gut bewachsenen Schrägröhrchen wurden 250 ml Flüssignährlösung (im 1 Lit. Erlenmeyerkolben) beimpft, die pro 1 l 10 g tryptisch verdautes Casein-Pepton, 5 g Hefeextrakt, 5 g Sorbit, 5 g NaCl und 10 g 1-tert.-Butyloxycarbonylaminosorbit enthielt. Die Nährbodenbestandteile wurden in entmineralisiertem Wasser gelöst und durch 20-minütiges Erhitzen im Autoklaven auf 121 °C sterilisiert. Die Kultur wurde bei 37 °C auf einer Rundschüttelmaschine bei 200 Upm inkubiert.

Der Gehalt an 1-tert.-Butyloxycarbonylaminosorbose wurde dünnschichtchromatographisch bestimmt. Nach 2 Tagen waren 5 g/l (entsprechend 50 %) umgesetzt. Zu diesem Zeitpunkt wurde die Fermentation abgebrochen.

## Beispiel 7

Oxidation von 1-tert.-Butyloxycarbonylamino-N-methylsorbit durch Glucobacter oxydans ssp. suboxydans (DSM 2003)

Eine Vorkultur von Glucobacter oxydans ssp. suboxydans (DSM 2003) wurde in folgender Nährlösung gezüchtet : 200 g/l Sorbit, 40 g/l Hefeextrakt, 10 g/l $KH_2PO_4$, gelöst in entmineralisiertem Wasser. Der pH-Wert wurde mit NaOH auf 6,5 eingestellt. 250 ml dieser Nährlösung, die für 20 Min. bei 121 °C autoklaviert worden war, wurde von einem Schrägröhrchen ausgehend beimpft und bei 28 °C auf einer Rundschüttelmaschine bei 280 Upm inkubiert. Nach 36 Stunden wurde mit dieser Vorkultur ein Fermenter beimpft, der 10 l Nährlösung folgender Zusammensetzung enthielt : 100 g/l Sorbit, 20 g/l Hefeextrakt, 5 g/l $KH_2PO_4$, 1 ml Polyol-Entschäumer, gelöst in entmineralisiertem Wasser. Der Fermenterinhalt wurde 30 Minuten bei 121 °C sterilisiert. Die Fermentationsbedingungen waren : Rührung : 500 Upm, Temperatur : 30 °C, Belüftung : 10 l Luft/Min. ; der pH-Wert wurde durch automatische Zugabe von 5 N NaOH bei 5,8 konstant gehalten.

Nach 36 Stunden wurden 500 ml einer 20 %igen, 70 °C heißen Lösung von 1-tert.-Butyloxycarbonylamino-N-methylsorbit steril zugegeben. Nach 84 Stunden war vollständiger Umsatz von 10 g/l erzielt. Die Isolierung des Oxidationsproduktes erfolgte analog Beispiel 4.

## Beispiel 8

Oxidation von 1-tert.-Butyloxycarbonylamino-N-(β-hydroxyethyl)-sorbit durch Glucobacter oxydans ssp. suboxydans (DSM 2003)

Glucobacter oxydans ssp. suboxydans (DSM 2003) wurde wie in Beispiel 7 beschrieben in einem 10 l Fermenter kultiviert. Nach 36 Stunden wurden 250 ml steril in einen 1 Lit. Erlenmeyerkolben abgefüllt, der 2,5 g steril eingewogenes 1-tert.-Butyloxycarbonylamino-N-(β-hydroxyethyl)-sorbit enthielt. Der Kolben wurde bei 28 °C und 280 Upm inkubiert. Nach 72 Stunden war vollständiger Umsatz erzielt. Die Isolierung des Oxidationsproduktes erfolgte analog Beispiel 4 mit n-Butanol.

## Beispiel 9

6-Amino-6-dexosy-L-sorbose-Hydrochlorid

20 g N-tert.-Butyloxycarbonyl-6-amino-6-desoxy-L-sorbose werden unter Rühren und Kühlen portionsweise in eine Mischung von 6,4 ml $H_2O$ und 13,2 ml konz. Salzsäure gegeben. Der Ansatz wird 2 Stunden ohne Kühlung nachgerührt und dann innerhalb von 2 Stunden mit 120 ml n-Propanol versetzt. Die entstandene Kristallsuspension wird jetzt 1 Stunde bei 0 °C gerührt und auf einer Nutsche abgesaugt. Das Kristallisat wird mit etwas n-Propanol gewaschen und bei Raumtemperatur im Vakuumtrockenschrank getrocknet.

Ausbeute : 14,2 g ≙ 92 % d.Th.
Schmelzpunkt (Zers.) : 116-125 °C.
$C_6H_{13}NO_5 \times HCl$ (215,2)
Ber. C 33,30 % H 6,54 % N 6,53 %
Gef. C 33,25 % H 6,68 % N 6,46 %

Das Produkt kann auch als Monohydrat kristallisieren. Schmelzpunkt (Zers.) : 70-75 °C auch dieses Material lieferte befriedigende Analysenwerte.

## Beispiel 10

Desoxynojirimycin-Hydrochlorid

100 g (0,47 Mol) 6-Amino-6-desoxy-L-sorbose-Hydrochlorid werden in 1 000 ml Wasser gelöst, mit 15 g Raney-Nickel versetzt und 3 Stunden bei Raumtemperatur und einem $H_2$-Druck von $20 \times 10^5$ Pa hydriert. Der Autoklav wird entspannt, der Kontakt wird abfiltriert und die resultierende Lösung wird im Vakuum eingedampft. Der feste Rückstand wird in der Hitze mit 40 ml Wasser gelöst und dann langsam bei 50 °C mit 400 ml Ethanol versetzt. Die gebildete Kristallsuspension wird auf − 5 °C abgekühlt, abgesaugt und mit etwas Ethanol gewaschen. Das Kristallisat wird über Nacht im Vakuumtrockenschrank bei 50 °C getrocknet.
Ausbeute : 70,5 g ≙ 75 %
Schmelzpunkt : 208-210 °C
Das so erhaltene Material war identisch mit einer Originalprobe (IR, HPLC).

## Beispiel 11

6-Methylamino-6-desoxy-L-sorbose-Hydrochlorid

Analog Beispiel 9 wurden 8 g (0,027 Mol) N-tert.-Butyloxycarbonyl-6-methylamino-6-desoxy-L-sorbose hydrolytisch deblockiert. Man erhält 5 g des sirupösen Hydrochlorids, das wegen der Zersetzlichkeit gleich weiter verarbeitet wurde.
Ausbeute : 5 g = 80 % Sirup.

## Beispiel 12

N-Methyl-1-desoxynojirimycin

5 g 6-Methylamino-6-desoxy-L-sorbose-Hydrochlorid werden in 50 ml $H_2O$ gelöst, mit 0,5 g 5 %iger Pt-Kohle versetzt und 3 Stunden bei Raumtemperatur und $20.10^5$ Pa $H_2$-Druck hydriert. Die filtrierte wäßrige Lösung des Hydrochlorids wird mit stark basischem Ionenaustauscher $Cl^-$ frei gemacht, zur Trockne eingedampft und aus Ethanol/$H_2O$ 10 : 1 kristallisiert.
Man erhält 2,3 g des krist. Produkts, ≙ 60 % d. Th. Schmelzpunkt : 152-154 °C.

## Beispiel 13

6-Hydroxyethylamino-L-sorbose-Hydrochlorid

Analog Beispiel 9 wurden 4 g (0,012 Mol) der BOC-geschützten 6-Hydroxyethylamino-L-sorbose mit 8 n Salzsäure umgesetzt und man erhielt 2,2 g 6-Hydroxyethylamino-L-sorbose-Hydrochlorid als Sirup. Das Produkt wurde nicht weiter charakterisiert, sondern sofort zur Hydrierung eingesetzt.

## Beispiel 14

N-Hydroxyethyl-1-Desoxynojirimycin

2,2 g 6-Hydroxyethylamino-L-sorbose-Hydrochlorid wurden in 30 ml Wasser gelöst, mit 300 mg 5 %iger Pt-Kohle versetzt und 3 Stunden bei Raumtemperatur und $20.10^5$ Pa $H_2$-Druck hydriert. Der Katalysator wurde abfiltriert und das wäßrige Filtrat wurde mit stark basischem Ionenaustauscher chloridfrei gemacht. Die wäßrige Lösung wurde am Rotationsverdampfer eingedampft und aus Ethanol/$H_2O$ 10 : 1 kristallisiert.
Ausbeute : 1 g ≙ 55 % d. Th.
Schmelzpunkt : 144-146 °C.

**Ansprüche**

1. Verfahren zur Herstellung von Verbindungen der Formel I

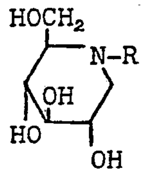

(I)

worin

R Wasserstoff oder gegebenenfalls substituiertes Alkyl oder Aralkyl bedeutet,

dadurch gekennzeichnet daß man Glucose in an sich bekannter Weise in 1-Aminosorbite der Formel IV

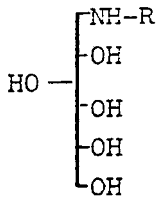

(IV)

worin

R die genannte Bedeutung hat,

überführt, die Aminogruppe mit einer sauer abspaltbaren und im sich anschließenden mikrobiologischen Oxidationsverfahren beständigen Schutzgruppe schützt, die so erhaltenen Verbindungen auf an sich bekannte Weise mikrobiologisch zu den geschützten 6-Aminosorbosen oxidiert, anschließend die Schutzgruppe sauer abspaltet und die so erhaltenen Salze der 6-Aminosorbosen entweder nach ihrer Isolierung oder in einem Arbeitsgang in an sich bekannter Weise zu den Verbindungen der Formel I hydriert.

2. Verfahren nach Anspruch 1, dadurch gekennzeichnet, daß der Rest R Wasserstoff, $C_1$-$C_{10}$-Alkyl oder Hydroxyethyl bedeutet.

3. Verfahren nach den Ansprüchen 1-2, dadurch gekennzeichnet, daß die Schutzgruppe tert.-Butyloxycarbonyl oder o-Nitrosulfenyl ist.

4. Verfahren zur Herstellung einer Verbindung der Formel VII

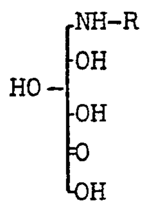

(VII)

in der R die in den Ansprüchen 1-3 angegebene Bedeutung hat, dadurch gekennzeichnet, daß man eine Verbindung der Formel XII

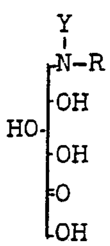

(XII)

in der R die obengenannte Bedeutung hat und Y eine Schutzgruppe darstellt mit einer Säure behandelt, die geeignet ist, die Schutzgruppe abzuspalten.

**Claims**

1. Process for the preparation of compounds of the formula I

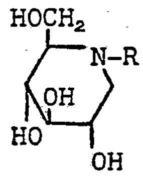

(I)

wherein

R denotes hydrogen or optionally substituted alkyl or aralkyl,

characterised in that glucose is converted into 1-aminosorbitols of the formula IV

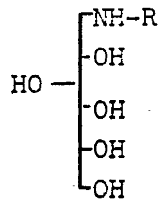

(IV)

wherein

R has the meaning given,

in a manner which is in itself known, the amino group is protected with a protective group which can be split off under acid conditions and is stable in the subsequent microbiological oxidation process, the compounds thus obtained are oxidised microbiologically in a manner which is in itself known to give the protected 6-aminosorboses, the protective group is then split off under acid conditions and the 6-aminosorbose salts thus obtained are hydrogenated in a manner which is in itself known, either after being isolated or in one operation, to give the compounds of the formula I.

2. Process according to Claim 1, characterised in that the radical R denotes hydrogen, $C_1$-$C_{10}$-alkyl or hydroxyethyl.

3. Process according to Claims 1-2, characterised in that the protective group is tert.-butoxycarbonyl or o-nitrosulphenyl.

4. Process for the preparation of a compound of the formula VII

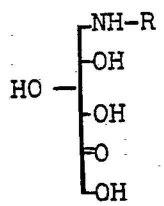

(VII)

in which R has the meaning given in Claims 1-3, characterised in that a compound of the formula XII

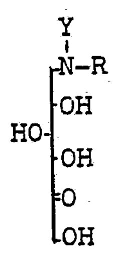

(XII)

in which R has the abovementioned meaning and Y represents a protective group, is treated with an acid which is suitable for splitting off the protective group.

**Revendications**

1. Procédé de production de composés de formule I

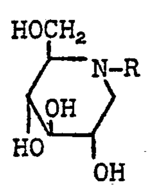

(I)

dans laquelle

R représente l'hydrogène ou un groupe alkyle ou aralkyle éventuellement substitué, caractérisé en ce qu'on transforme du glucose d'une manière connue en 1-aminosorbitols de formule IV

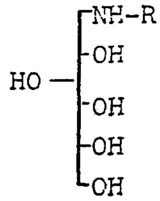

(IV)

dans laquelle

R a la définition indiquée ci-dessus,

on protège le groupe amino avec un groupe protecteur éliminable par voie acide et stable au cours du processus d'oxydation microbiologique subséquent, on oxyde les composés ainsi obtenus d'une manière connue par voie microbiologique en les 6-aminosorboses protégés, on élimine ensuite le groupe protecteur par voie acide et on hydrogène les sels, ainsi obtenus, des 6-aminosorboses soit après leur isolement, soit en une seule phase de travail d'une manière connue en les composés de formule I.

2. Procédé suivant la revendication 1, caractérisé en ce que le reste R représente l'hydrogène, un reste alkyle en $C_1$ à $C_{10}$ ou le reste hydroxyéthyle.

3. Procédé suivant les revendications 1 et 2, caractérisé en ce que le groupe protecteur est un groupe tertio-butyloxycarbonyle ou o-nitrosulfényle.

4. Procédé de production d'un composé de formule VII

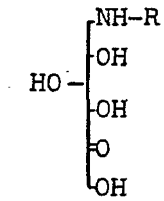

(VII)

dans laquelle R a les définitions indiquées dans les revendications 1 à 3, caractérisé en ce qu'on traite un composé de formule XII

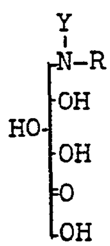

(XII)

dans laquelle R a la définition indiquée ci-dessus et Y représente un groupe protecteur, avec un acide qui convient pour éliminer le groupe protecteur.